# EUROPÄISCHE PATENTSCHRIFT

(11) **EP 1 337 183 B1**
(45) Veröffentlichungstag und Bekanntmachung des Hinweises auf die Patenterteilung: **25.05.2005**
(21) Anmeldenummer: 01995652.3
(22) Anmeldetag: 21.11.2001
(51) Int. Cl.: A61B 8/12

(54) **ULTRASCHALLSONDE MIT POSITIONIEREINRICHTUNG FÜR UNTERSUCHUNGS- UND OPERATIONSVORRICHTUNGEN**
ULTRASONIC PROBE COMPRISING A POSITIONING DEVICE FOR EXAMINATION DEVICES AND OPERATION DEVICES
SONDE A ULTRASONS DOTEE D'UN SYSTEME DE POSITIONNEMENT DESTINE A DES DISPOSITIFS D'EXPLORATION ET D'INTERVENTION

(30) Priorität: 24.11.2000 DE 10058370; 18.07.2001 DE 10134911
(43) Veröffentlichungstag der Anmeldung: 27.08.2003
(73) Patentinhaber: Innovacell Biotechnologie GmbH, 6020 Innsbruck (AT)
(72) Erfinder: Hering, Steffen, A-6020 Innsbruck (AT); Strasser, Hannes, A-6060 Hall (AT); Marksteiner, Rainer, A-6360 Schwaz (AT)
(74) Vertreter: Hertz, Oliver, Dr.
(86) Internationale Anmeldenummer: PCT/EP2001/013535
(87) Internationale Veröffentlichungsnummer: WO 2002/045588

(56) Entgegenhaltungen:
- EP-A- 0 446 645
- WO-A-96/32889
- JP-A- 11 244 289
- US-A- 4 763 662
- US-A- 4 869 258
- US-A- 4 877 033
- US-A- 4 883 059
- US-A- 4 911 173
- US-A- 5 235 987
- US-B1- 6 238 336

## Beschreibung

Die Erfindung betrifft eine endoluminale Ultraschallsonde, die mit einer Positioniereinrichtung ausgestattet ist, die insbesondere zur Führung und Positionierung einer weiteren Untersuchungs- oder Operationsvorrichtung im Körper des Organismus eingerichtet ist. Es werden auch ein Verfahren zur Ausrichtung und/oder Betätigung von Untersuchungs- oder Operationsvorrichtungen im Körper und Verwendungen von Untersuchungssonden, insbesondere einer endoluminalen Ultraschallsonde, mit einer Positioniereinrichtung beschrieben.

Moderne Ultraschallgeräte liefern einen wichtigen Beitrag zur bildgebenden Diagnostik und ermöglichen eine schnelle, schmerz- und gefahrlose Untersuchung von Organen und anatomischen sowie pathologischen Strukturen. Darüber hinaus wurden sonographische Techniken entwickelt, mit deren Hilfe es möglich ist, ultraschallgezielt Biopsien oder Aspirate aus Organen oder Körperhöhlen zu entnehmen.

Bereits Anfang der 80-er Jahre wurden erste Punktionshilfen für Schallsonden beschrieben (siehe z. B. US 4363326, US 4402324, US 4489730, US 4542747 und US 4635644). Bei diesen Vorrichtungen handelt es sich um einfache Punktionsführungen für Ultraschallsonden, mit denen von der Körperoberfläche aus ultraschallgezielt Biopsien aus dem Körper entnommen werden konnten. Es sind auch Punktionsführungen bekannt, die einerseits fix in die Ultraschallsonde integriert (US 4363326 und US 4635644) oder abnehmbar sind (US 4402324). Diese abnehmbaren Punktionshilfen weisen unterschiedliche Formen auf. So wurde unter anderem eine keilförmige Punktionsführung entwickelt, die in eine genau passende keilförmige seitliche Aussparung in der Ultraschallsonde passt (US 4489730). Nach der Punktion kann die Punktionsnadel mitsamt der Punktionsführung aus der Ultraschallsonde entnommen werden, so dass die Nadel im Körper des Patienten belassen werden und die Ultraschallsonde gleichzeitig entfernt werden konnte. In US 4542747 wird eine Ultraschallsonde beschrieben, die aus 2 Hälften mit je einem Führungsschlitz besteht. Nach dem Zusammenfügen der beiden Hälften entsteht durch die beiden zusammenpassenden Schlitze ein Punktionskanal. Nach der Punktion können die beiden Ultraschallsonden-Hälften wieder auseinandergenommen und entfernt werden, so dass wiederum die Nadel frei im Patienten belassen werden kann. Aus US 5052396 ist eine abnehmbare Punktionsführung bekannt, die mehrere Führungsschlitze zur Aufnahme von Nadeln mit unterschiedlichen Durchmessern aufweist.

Es sind auch Punktionshilfen für endoluminale Ultraschallsonden bekannt. So ist eine transvaginale Ultraschallsonde bekannt, auf die eine Punktionsführung seitlich aufgesetzt wurde, so dass ultraschallgezielt Eizellen aus den Ovarien oder Biopsien aus den weiblichen Beckenorganen entfernt werden konnten (US 4742829). Zur verbesserten Darstellung der Punktionsnadel im Ultraschallbild wurde neben normalen Sonographie-Techniken auch ein Vibratiönssystem entwickelt, das die Punktionsnadel in feine Schwingungen versetzt, wodurch die Nadel aufgrund des Doppler-Effekts besser dargestellt werden kann (US 5343865). Darüber hinaus sind auch biegbare endoluminale Ultraschallsonden mit einem Arbeitskanal bekannt, der nicht durch das Abbiegen der Ultraschallsonde beeinträchtigt wird (US 5469853). Mit diesem System können Biopsien im Rahmen von endochirurgischen Operationen entnommen werden. Es werden auch chirurgische Instrumente eingesetzt, bei denen vor dem Schneiden oder Koagulieren mit dem Schallkopf des Gerätes das zu behandelnde Gewebe vorab untersucht werden kann.

In den letzten Jahren wurden die genannten Formen der Punktionshilfen noch vereinfacht und verbessert. So sind schalenförmige Führungen bekannt, die den Durchmesser der Ultraschallsonde nur geringfügig vergrößern (US 5924992). Außerdem sind Haltesysteme der Punktionsführungen bekannt, die bei endoluminalen Ultraschallsonden ein Abnehmen der eingeführten Nadel von der Schallsonde ermöglichen, so dass die Ultraschallsonde entfernt und die Punktionsnadel im Patienten belassen werden kann (US 6095981).

Die herkömmlichen Ultraschallsonden mit Punktionshilfen besitzen eine Reihe von Nachteilen. Die Funktion der bekannten Anordnungen wird dadurch eingeschränkt, dass der Abstand einer eingeführten Diagnostik- oder Operationsvorrichtung (z. B. einer Nadel) vom Schallkopf fixiert ist und weder verändert noch im Ultraschallbild exakt kontrolliert werden kann. Keines der beschriebenen Punktionssysteme für Ultraschallsonden ist so steuerbar, dass der Abstand von eingeführten Arbeitsgeräten zum Schallkopf bei vorgegebener Lage der endoluminalen Schallsonde im Patienten manipulierbar ist. Es ist daher mit den vorhandenen Ultraschallsonden und den mit ihnen verbundenen Punktionssystemen unmöglich, Katheter, Punktionsnadeln oder andere Untersuchungs- oder Operationsvorrichtungen mit Hilfe einer endoluminalen Schallsonde gezielt und in variablem Abstand vom Schallkopf im umgebenden Gewebe von Gefäßen oder engen Hohlorganen zu platzieren, da die Lage der Ultraschallsonde so manipuliert werden müsste, dass das Hohlorgan oder das Gefäß, in dem sich die Sonde befindet, verletzt (z. B. eingerissen, abgerissen, gequetscht, perforiert) würden, oder weil diese Manipulation durch Umgebungsstrukturen zu sehr behindert wird. Eine endoluminale Ultraschallsonde kann in diesen Fällen nur in der Längsachse verschoben oder um diese rotiert werden.

Mit den im Stand der Technik beschriebenen Ultraschallsonden mit Punktionshilfen ist die genaue Punktion einer Struktur, die sich seitlich im Wandbereich eines engen Lumens (z. B. der Harnröhre oder eines Blutgefäßes) befindet, wegen der fehlenden Veränderbarkeit des Austrittswinkels der Diagnostik- oder Operationsvorrichtung aus der Punktionsvorrichtung nicht möglich. Darüber hinaus können die Punktionsführungen bisher nicht gezielt entlang des Schaftes der endoluminalen Schallsonden verschoben werden. Diese Nachteile gelten auch für herkömmliche endoluminale MRI-Sonden.

Es ist ferner allgemein bekannt, dass die Inspektion der Harnröhre und die Injektion von Füllstoffen ("bulking agents") bisher vorrangig mit Hilfe von Endoskopen erfolgt. Um Verletzungen beim Einführen des Endoskops (besonders der auskleidenden Schleimhäute und der darunter liegenden Schichten) zu vermeiden, sind derartige Endoskope in der Regel mit einem Perfusionskanal ausgestattet, der an der Vorderseite des Endoskops austritt. Durch das kontinuierliche Spülen der Harnröhre mit Flüssigkeit wird die Harnröhre vor der Endoskoplinse geweitet. Dadurch kann das Endoskop eingeführt werden, ohne dass Perforationen der Harnröhrenwand auftreten. Der Verlauf und das Lumen der Harnröhre werden durch das Aufweiten vor dem Endoskop in ihrem Verlauf einsehbar. Gleichzeitig kann durch Applikation von Flüssigkeit in die Harnröhre während der Therapie der Therapieerfolg festgestellt werden.

Herkömmliche Konstruktionen transluminaler Schallsonden, sind für die Applikation von Füllstoffen in die Urethra nur bedingt verwendbar, da beim Einführen der Sonde in die Harnröhre durch ungeübte Operateure Verletzungen auftreten können. Bei transurethralen Schallsonden mit nur einer Schallebene (d.h. Sonden, die nur die Darstellung des transversalen (Querschnitt) Bildes der Röhre gestatten) ist deshalb häufig für die gefahrlose Einführung der Sonde in die Urethra zusätzlich eine Endoskopvorrichtung erforderlich.

Durch das Einführen einer zweiten Schallebene in den Schallkopf einer transluminalen Schallsonde wird, neben dem Erfassen des Querschnittes der Harnröhre, zusätzlich ein longitudinales Schnittbild der Harnröhre erzeugt und damit die longitudinale Darstellung der Harnröhre (d.h. in ihrem Längsverlauf) mit einer endoluminalen Schallsonde möglich. Derartige Sonden haben jedoch den Nachteil, dass sie beim Einführen in das Lumen der Harnröhre unmittelbar an ihrer Innenwand anliegen und dem Operateur die longitudinale Einsicht in das Lumen der Harnröhre verwehrt ist. Da die Harnröhre nicht mit Flüssigkeit gefüllt ist, kann es so beim Einführen der Sonde (besonders beim Mann) zu schweren Verletzungen kommen.

Bei transrektalen Schallsonden sind Ballonkonstruktionen in der Nähe des Schallkopfes bekannt, die zum Aufweiten des Rektums gefüllt werden, wodurch die Einsicht in das Lumen verbessert wird. Diese Ausführungsformen von transluminalen Sonden sind für Untersuchungen der besonders engen Harnröhre jedoch nicht geeignet, da sie keinen Perfusionskanal zum Aufweiten des Lumens durch einen Spülvorgang des Kanals aufweisen.

Ein weiterer Nachteil herkömmlicher Konstruktionen von Ultraschallsonden mit Manipulatoren ist durch deren komplizierten Aufbau gegeben. Herkömmliche Manipulatoren sind mit einer Mikromechanik ausgestattet und daher in der Herstellung kostenaufwendig und komplex. Eine Einwegnutzung ist mit mikromechanischen Manipulatoren nicht praktikabel.

Es sind auch flexible Ultraschallsonden und Endoskopvorrichtungen bekannt, die mit Ablenkvorrichtungen ausgestattet sind. Eine herkömmliche Ablenkvorrichtung wird beispielsweise durch einen Seilzug bereitgestellt, der in den Sondenschacht integriert ist und mit dessen Hilfe der Ultraschallkopf ablenkbar ist.

In US 4 911 173 wird eine Ultraschallsonde gemäß dem Oberbegriff der Anspruchs 1 offenbart. Aus EP 446 645 ist eine Ultraschallsonde mit einem relativ zur Achse eines Schaftes abgebogenen Ultraschallkopf bekannt. In WO 96/32889 wird die Einführung einer Nadelführung in einen Organismus für die Aufnahme von Biopsieproben beschrieben.

Die Aufgabe der Erfindung ist es, eine verbesserte Ultraschallsonde mit einer Positioniereinrichtung anzugeben, mit der die Nachteile der herkömmlichen Anordnungen überwunden werden und die insbesondere einen erweiterten Anwendungsbereich besitzt. Mit der Erfindung soll insbesondere eine präzise Überwachung der Positionierung von Diagnose- oder Operationsvorrichtungen im Körper eines Organismus ermöglicht werden.

Diese Aufgabe wird durch Vorrichtungen mit den Merkmalen gemäß den Patentansprüchen 1 und 12 gelöst. Vorteilhafte Ausführungsformen der Erfindung ergeben sich aus den abhängigen Ansprüchen.

Es wird insbesondere vorgeschlagen, bei einer Ultraschallsonde mit einem Ultraschallkopf, der an einem starren Schaft befestigt und mit diesem beweglich ist, eine Positioniereinrichtung für mindestens eine Untersuchungs- und Operationsvorrichtung vorzusehen, wobei die Positioniereinrichtung mindestens eine Punktionsführung aufweist, die eine Führung mit einer definierten Halterung oder einem Anschlag für die Untersuchungs- oder Operationsvorrichtung bildet, und die Untersuchungs- und Operationsvorrichtung mit einer Ablenkvorrichtung derart verstellbar ist, dass der Anschlag (oder ein Lumen der Punktionsführung, eine Austrittsöffnung des Lumens oder die im Betriebszustand in das Lumen eingeführte Untersuchungs- oder Operationsvorrichtung) eine vorbestimmte, ggf. zeitlich veränderliche, Position relativ zum Ultraschallkopf besitzt. Die Ultraschallsonde ist vorzugsweise eine endoluminale Ultraschallsonde. Die Untersuchungs- oder Operationsvorrichtung (im Folgenden: Manipulationsvorrichtung) ist bspw. eine Biopsieeinrichtung, eine Injektionspipette oder ein chirurgisches Gerät.

Die erfindungsgemäße Ultraschallsonde besitzt den Vorteil, dass die Positioniereinrichtung für die Manipulationsvorrichtung gleichzeitig eine definierte Halterung bildet und eine flexible Verstellbarkeit (Verschiebbarkeit, Winkeleinstellung) in bestimmte Positionen ermöglicht.

Mit der Ultraschallsonde mit flexibler Punktionshilfe ist es erstmals möglich, den Abstand zwischen dem Schallkopf und der eingeführten Manipulationsvorrichtung, die z. B. seitlich der Ultraschallsonde aus der Führungsvorrichtung austritt, gezielt zu verändern. Diese Eigenschaft bringt entscheidende Vorteile mit sich, besonders bei Eingriffen in Hohlorganen, Gefäßen, Gelenken, anderen Körperhöhlen oder engen Operationsgebieten, bei denen die Ultraschallsonde praktisch nur gedreht, vorgeschoben oder zurückgezogen werden kann. Durch den Einsatz einer endoluminalen Ultraschallsonde mit flexibler Punktionshilfe kann die Lage der Operations- oder Diagnosevorrichtung (z. B. eines Katheters) erstmals gezielt während der Untersuchung oder Operation verändert werden.

Gemäß einer bevorzugten Ausführungsform der Erfindung ist die Ablenkvorrichtung für die mindestens eine Manipulationsvorrichtung mit einer Steuervorrichtung verstellbar. Die Ablenkvorrichtung wird so betrieben, dass der Anschlag der Punktionsführung oder die eingeführte Manipulationsvorrichtung eine bestimmte Position relativ zum Ultraschallkopf besitzt oder entlang einer bestimmten Wegstrecke relativ zum Ultraschallkopf bewegt wird. Die Position wird durch bestimmte Raumkoordinaten relativ zum Ultraschallkopf repräsentiert. Die Steuervorrichtung ist vorzugsweise über einen Koordinatengeber mit dem bildgebenden System der Ultraschallsonde verbunden. Dieses enthält insbesondere einen Anzeigebildschirm zur Anzeige von Ultraschallbildern. Es ist erfindungsgemäß vorgesehen, dass mit dem Koordinatengeber der Steuervorrichtung Signale entsprechend den eingestellten Relativkoordinaten der Punktionsführung an das bildgebende System übertragen und zur Anzeige einer Markierung, z. B. einer Punktionshilfslinie oder eines Punktionshilfspunktes, auf der Anzeigeeinrichtung verwendet werden.

Eine Ausführungsform der Erfindung ist somit auch eine Ultraschallsonde mit einem bildgebenden System, das mit der Steuervorrichtung einer Punktionsführung verbunden und dazu eingerichtet ist, die Position der Punktionsführung oder der in diese eingeführten Manipulationsvorrichtung auf einer Anzeigeeinrichtung anzugeben. Zur Steuerung einer Positioniereinrichtung einer Ultraschallsonde wird die aktuelle Ausrichtung und/oder Bewegung einer Positioniereinrichtung relativ zum Ultraschallkopf auf der Anzeigeeinrichtung des bildgebenden Systems abgebildet.

Die Punktionshilfsmarkierung auf der Anzeigeeinrichtung (bspw. eine Punktionshilfslinie oder ein Punktionshilfspunkt) bietet die Möglichkeit, vor dem Austritt der Manipulationsvorrichtungen oder Arbeitsgeräte aus dem Arbeitskanal (Lumen) der Punktionsführung die exakte Endlage im Ultraschallbild vorauszuberechnen und die Punktion z. B. entlang der vorausberechneten Punktionshilfslinie im Ultraschallbild auch zu verfolgen. Nach dem Vorschieben der jeweiligen Arbeitsgeräte kann dann im Echtzeit-Ultraschallbild deren Lage aktuell kontrolliert und die Applikation verfolgt werden. Neben mono- oder biplanaren Standard-Ultraschallsonden können auch neuere Ultraschalltechniken, wie z. B. 3D-Sonographie oder Farbdoppler-Sonographie, verwendet werden. Mit Hilfe der verschiedensten Diagnose- oder Operationsvorrichtungen können unter anderem Biopsien entnommen, Medikamente, Lösungen oder Zellsuspensionen injiziert, über verschiedenen Arbeitssonden Energie appliziert oder chirurgische Geräte eingeführt und gezielt platziert und betätigt werden.

Gemäß einer weiteren Ausführungsform der Erfindung ist eine endoluminale Ultraschallsonde vorgesehen, bei der die Diagnose- oder Operationsvorrichtung in der Ultraschallsonde und durch den Ultraschallkopf verläuft, so dass mit Hilfe der Ablenkvorrichtung der Ultraschallkopf und die Diagnose- oder Operationsvorrichtung gleichzeitig gezielt abgelenkt werden können. Diese Variante stellt insbesondere bei laparoskopischen Ultraschallsonden einen bedeutenden Fortschritt dar.

Die Position einer Manipulationsvorrichtung, die in die Punktionsführung-einführbar ist, kann vor dem Austritt aus der Punktionsführung berechnet werden. Die errechenbare Position der Manipulationsvorrichtung nach Austritt aus der Punktionsführung wird im Ultraschallbild mit Hilfe einer Punktionshilfslinie oder eines Punktionshilfspunktes angezeigt. Mit Hilfe des Koordinatengebers können die Punktionshilfslinie bzw. der Punktionshilfspunkt gleichzeitig mit der Ablenk- oder Manipulationseinrichtung der Punktionsführung verändert und deren Lage koordiniert ausgewählt werden.

Durch die steuerbare Ablenkvorrichtung der Punktionsführung der Ultraschallsonde können z. B. Injektionsnadeln sehr präzise in Hohlorganen, Gefäßen, Gelenken und Körperhöhlen platziert werden. Dadurch ist erstmals eine exakte und steuerbare Injektion von Medikamenten, Zellen oder therapeutischen Substanzen in den Körper des Patienten möglich, wobei der Ort der Applikation der Untersuchungs- oder Operationsvorrichtung verändert und vor dem eigentlichen Eingriff exakt geplant und ausgewählt werden kann. Es können mit dieser Ultraschallsonde alle Arten von Untersuchungs- oder Operationsvorrichtungen ultraschallgezielt durch ein Arbeitslumen der Punktionsvorrichtung in den Körper des Patienten eingeführt werden, wobei diese Technologie alle Vorteile der minimal invasiven Chirurgie aufweist.

Gemäß einer weiteren Ausführungsform der Erfindung ist eine endoluminale Ultraschallsonde mit einer biplanaren StandardUltraschallsonde vorgesehen, die zusätzlich zu den oben beschriebenen Injektionsvorrichtungen einen Perfusionskanal oder einen abnehmbaren Perfusionskatheder aufweist. Bei dieser Ausführungsform wird vorteilhafterweise die Erfassung von longitudinalen Schallbildern ermöglicht.

Gemäß einer weiteren Ausgestaltung ist eine erfindungsgemäße Ultraschallsonde mit einem Drucksensor ausgestattet. Eine solche endoluminale Schallsonde gestattet nicht nur die ultraschallgezielte Injektion von Füllstoffen in die Wand bspw. der Harnröhre, sondern auch die Kontrolle des Urethradruckprofiles (und damit des Therapieerfolges) mit der selben Vorrichtung während der Therapie. Der Drucksensor umfasst beispielsweise einen an sich bekannten Druckmesser (z.B. mit einer Membran, die bei Druckveränderungen ausgelenkt wird und Druckänderungen z.B. über eine Flüssigkeitssäule auf einen Geber überträgt), mit dem ein Druckprofil in einem Lumen, z.B. der Harnröhre, bestimmt werden kann.

Mit der Erfindung wird auch ein medizinisches Gerät bereitgestellt, das die erfindungsgemäße Ultraschallsonde und mindestens eine Untersuchungs- oder Manipulationsvorrichtung umfasst.

Es wird auch ein Verfahren zur Untersuchung und/oder Behandlung von Gewebe oder Organen mit der erfindungsgemäßen Ultraschallsonde beschrieben. Dies kann mit oder ohne Verwendung der Punktionshilfsmarkierung auf der Anzeigevorrichtung erfolgen.

Bei dem Verfahren wird die Ultraschallsonde zunächst anwendungsabhängig an oder in dem interessierenden Gewebe oder Organ positioniert wird. Daraufhin erfolgt eine Betätigung der Positioniereinrichtung derart, dass die Punktionsführung bestimmte Relativkoordinaten relativ zum Ultraschallkopf einnimmt. Dann wird die jeweilige Manipulationsvorrichtung (Untersuchungs- oder Operationsvorrichtung) in die Positioniereinrichtung eingeführt und bis zum Anschlag bewegt und in dieser Betriebsposition betätigt. In der Betriebsposition ist ggf. eine Bewegung der Manipulationsvorrichtung entlang der Punktionshilfsmarkierung vorgesehen. Diese Bewegung wird mit der Steuervorrichtung gesteuert.

Wichtige Schritte des Verfahrens zur Positionierung von Untersuchungs- oder Operationseinrichtungen bestehen in der Platzierung der Sonde im Körper mit einer Erfassung des zu untersuchenden oder zu operierenden Gewebes, die Ausrichtung der Positioniereinrichtung und die Einführung und ggf. Betätigung der Untersuchungs- oder Operationseinrichtung.

Es wird auch ein Verfahren zur Injektion von lebenden Zellen in einem Trägermedium und/oder Biomaterialien oder biokompatiblen Substanzen in einen Organismus beschrieben, wobei die erfindungsgemäße Ultraschallsonde verwendet wird.

Die Erfindung wird unter Bezug auf eine Sonde mit einem Ultraschallkopf beschrieben, ist aber auf diese Anwendung nicht beschränkt. Alternativ kann die Sonde bei allen beschriebenen Ausführungsformen auch mit einem sog. MRI-Kopf ausgestattet sein, wie es zur Magnetresonanzabbildung (MRI, magnetic resonance imaging) an sich bekannt ist.

Einzelheiten und weitere Vorteile der Erfindung werden im folgenden unter Bezug auf die beigefügten Zeichnungen beschrieben. Es zeigen:
- Figuren 1 bis 4: schematische, teilweise geschnittene Ansichten von Ultrasonden, wobei die in Figuren 3 und 4 gezeigten Ultraschallsonden keine Ausführungsformen der Erfindung sind.
- Figuren 5a, 5b: schematische Illustrationen der erfindungsgemäßen Anzeige von Punktionshilfsmarkierungen,
- Figuren 6 bis 8: weitere teilweise geschnittene Ansichten von Ultraschallsonden, und
- Figur 9: schematische Schnittansichten von Ausführungsformen erfindungsgemäßer medizinischer Arbeitsgeräte.

Die Erfindung repräsentiert ein steuerbares Ultraschall-Diagnostik- und Operations-Gerät, mit dem erstmals Diagnostik- und Operationsvorrichtungen ultraschallgezielt unter einem veränderbaren Winkel bezüglich der Achse der Schallsonde im Ultraschallkopf und in einem kalkulierbaren Abstand vom Schallkopf in Gewebe eingebracht werden können. Ein wesentlicher Vorteil der erfindungsgemäßen Konstruktion im Vergleich zu bisherigen Geräten besteht darin, dass bei vorgegebener Lage der Ultraschallsonde eine in die Punktionsführung eingeführte Manipulationsvorrichtung in beliebig wählbarem Abstand vom Schallkopf der Ultraschallsonde platziert werden kann. Die neuartige Punktionsführung wirkt mit mindestens einer Ablenkvorrichtung zusammen, die von einer Steuervorrichtung so manipuliert werden kann, dass die Punktionsführung und/oder eine in sie eingeführte Manipulationsvorrichtung abgelenkt oder entlang der Ultraschallsonde verschoben werden kann.

Gleichzeitig kann mit Hilfe einer mathematischen Kalkulation die Position der Manipulationsvorrichtung, der in die Punktionsführung einführbar ist, vor dem Austritt aus der Punktionsführung berechnet und die errechnete Endposition der Manipulationsvorrichtung vor Austritt aus der Punktionsführung in der Anzeige des Ultraschallbildes als Punktionshilfslinie oder Punktionshilfspunkt dargestellt werden. Somit können vor dem eigentlichen Eingriff vom Untersucher oder Operateur der Eintrittswinkel und die Endposition der Untersuchungs- oder Operationsvorrichtung vorausberechnet und mit der Steuervorrichtung während des Eingriffs ausgewählt werden. Die Applikation der Untersuchungs- oder Operationsvorrichtung (z. B. das Einführen einer Nadel) kann dann im Echtzeit-Ultraschallbild genau kontrolliert werden.

Die Erfindung wird im Folgenden unter Bezug auf bevorzugte Ausführungsformen beschrieben, bei denen die Ultraschallsonde einen Ultraschallkopf K besitzt, der am Ende eines Schaftes S befestigt und mit diesem manuell beweglich ist, wobei die Positioniereinrichtung P am oder im Schaft S angeordnet ist.

In den Figuren sind lediglich Einzelheiten der Positioniereinrichtung und der Ausbildung der Punktionshilfsmarkierung illustriert. Die Merkmale einer Ultraschallsonde mit einem Griff, einem Schaft und dem Ultraschallkopf sind an sich bekannt und werden daher ebenso wie Einzelheiten des bildgebenden Systems nicht beschrieben.

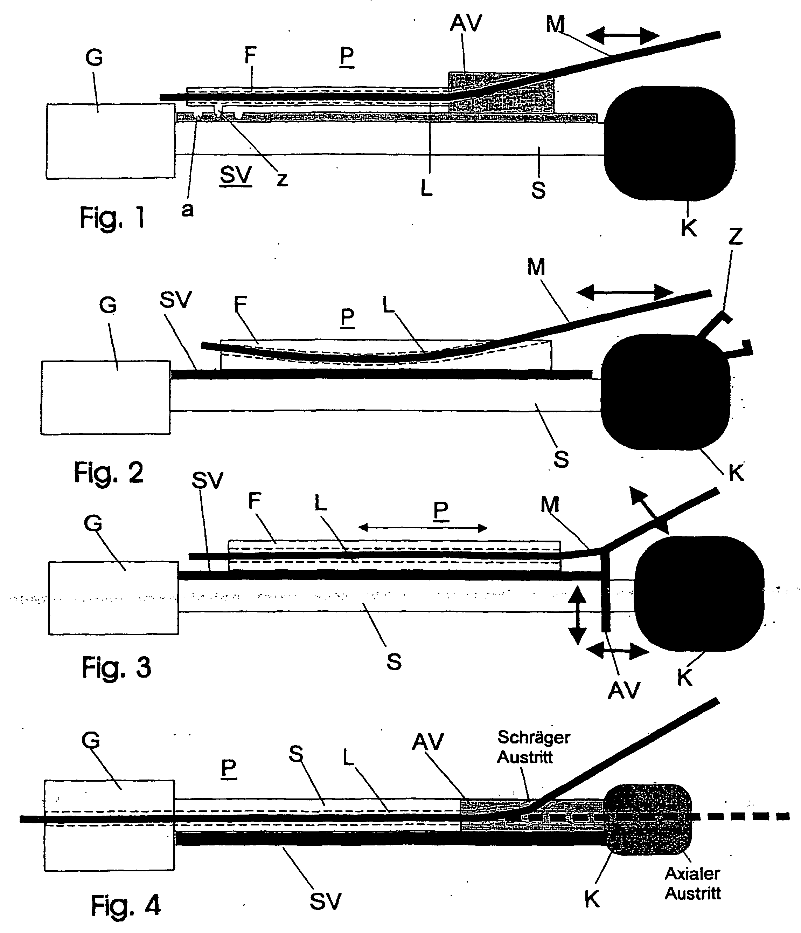

Eine erste Ausführungsform einer erfindungsgemäßen Ultraschallsonde mit dem Griff G, dem Schaft S und dem Schallkopf K ist in Figur 1 schematisch dargestellt. Die Positioniereinrichtung ist am Schaft S angebracht und wird durch die Punktionsführung F und die Ablenkvorrichtung AV gebildet. Die Punktionsführung enthält als Führung ein rohr- oder schlitzförmiges Lumen L, das als Arbeitskanal durch die Punktionsführung F und die Ablenkvorrichtung AV hindurchtritt. In der Ablenkvorrichtung AV ist der Arbeitskanal in seiner Ausrichtung relativ zur Sonde verstellbar. Die Punktionsführung F und die Ablenkvorrichtung AV bilden ein langgestrecktes Bauteil, das parallel zur Achse des Schaftes S verläuft. In der Ablenkvorrichtung AV ist ein vorbestimmter Winkel des Arbeitskanals oder Lumens relativ zur Schaftachse vorgegeben. Die Ablenkvorrichtung AV und die Punktionsführung F können mit Hilfe der Steuervorrichtung SV entlang des Schaftes S der Schallsonde gezielt verschoben werden. Dadurch ändert sich der Abstand der Manipulationsvorrichtung M, die sich im Arbeitslumen L befindet, vom Schallkopf K. Die Verschiebung kann diskret oder kontinuierlich arretierbar erfolgen. Die Steuervorrichtung besitzt beim dargestellten Ausführungsbeispiel zur diskreten Arretierung einen Zapfen z in der Punktionsführung F, der genau in eine von mehreren Aussparungen a der Schallsonde passt. Die Punktionsführung F ist flexibel, so dass sie leicht angehoben oder vom Schaft weggeschwenkt und mit der Ablenkvorrichtung verschoben und neu positioniert werden kann. Die Position der Punktionsführung F, der Ablenkvorrichtung AV und der eingeführten Manipulationsvorrichtung M korrelieren nach dem Einrasten des Zapfens der Punktionsführung F mit entsprechenden Punktionhilfslinien oder Punktionshilfspunkten im Ultraschallbild der Anzeigevorrichtung. Im Lumen L verläuft die Manipulationsvorrichtung M, die bspw. eine Punktionsnadel oder ein Katheter ist.

Zur kontinuierlichen Arretierung kann an Stelle der Zapfen eine geeignete lösbare Verbindung, z. B. ein Klemmring, vorgesehen sein.

Der Ultraschallkopf K ist in an sich bekannter Weise aufgebaut und enthält anwendungsabhängig einen oder mehrere Schallwandler (Transducer-Elemente) zur Aussendung bzw. zum Empfangen von Ultraschallwellen. Der Ultraschallkopf K kann zur Erzeugung uniplanarer oder biplanarer Echtzeit-Ultraschallbilder oder von Doppler-, Farbdoppler- oder 3D-Ultraschallbildern eingerichtet sein. Der Schaft S besteht aus einem starren Material. Die Punktionsführung F ist zumindest teilweise flexibel oder starr gestaltet. Im Falle einer endoluminalen Ultraschallsonde besitzt der Ultraschallkopf K typischerweise einen Durchmesser von ca. 5 bis 10 mm. Das Lumen L in der Ablenkvorrichtung AV besitzt einen relativ zur Längsachse des Schafts S gekrümmten Verlauf. Das zum Ultraschallkopf K weisende Ende des Lumens L bildet mit der Schaftachse einen bestimmten, festen Ablenkwinkel, durch den die Neigung der Manipulationsvorrichtung M relativ zur Schaftachse eingestellt ist. Die mit der Steuervorrichtung vermittelte Bewegung der Ablenkvorrichtung AV ermöglicht somit eine Verstellung der Manipulationsvorrichtung M entsprechend dem Doppelpfeil.

Die Punktionsführung ist mit dem Schaft der Ultraschallsonde fest verbunden, kann jedoch abgenommen und ausgetauscht werden.

Bei der in Figur 2 schematisch dargestellten Ausführungsform einer erfindungsgemäßen Ultraschallsonde mit dem Griff G, dem Schaft S und dem Ultraschallkopf K ist die Punktionsführung F wiederum seitlich mit dem Lumen L am Schaft S angebracht. Die Ablenkvorrichtung AV fällt bei dieser Ausführungsform mit der Punktionsführung F zusammen. Die Steuervorrichtung SV wird durch einen axialen Stellantrieb am Schaft S gebildet. Um eine axiale Bewegung der Manipulationsvorrichtung M entsprechend dem Doppelpfeil zu ermöglichen, ist die Punktionsführung F mit der Steuervorrichtung axial parallel zur Ausrichtung der Schaftachse verschiebbar, so dass die eingeführte Manipulationsvorrichtung mit verschoben wird. Dadurch ändert sich wiederum der Abstand der schräg aus der Punktionsführung austretenden Manipulationsvorrichtung vom Ultraschallkopf. Je nach Positionierung der Ablenkvorrichtung AV besitzt das Ende der Manipulationsvorrichtung M definierte Relativkoordinaten in Bezug auf den Ultraschallkopf K.

Durch eine definierte Einstellung der Ablenkvorrichtung AV und/oder Punktionsführung F wird die Manipulationsvorrichtung M so eingestellt, dass ihr Ende bestimmte Relativkoordinaten in Bezug auf den Ultraschallkopf besitzt. Dies setzt voraus, dass die Einschublänge der Manipulationsvorrichtung M bekannt oder definiert eingestellt ist. Zur Einstellung der Einschublänge ist vorzugsweise an der Punktionsführung oder in der Ablenkvorrichtung AV ein Anschlag (nicht dargestellt) vorgesehen, der mit einem entsprechenden Vorsprung an der Manipulationsvorrichtung M zusammenwirkt. Der Anschlag kann aber auch am Griff oder einem anderen Teil der Ultraschallsonde vorgesehen oder bei Verwendung von Markierungen an der Manipulationsvorrichtung M, mit denen sich die Einschublänge an der Ultraschallsonde feststellen lässt, ganz fortgelassen werden.

Der Ultraschallkopf kann mit einer Klemmeinrichtung zur zeitweiligen Fixierung am zu untersuchenden Gewebe ausgestattet sein. Die Klemmeinrichtung wird beispielsweise durch eine vom Griff her betätigbare Zange Z gebildet und ermöglicht, dass der Ultraschallkopf vor Einsetzen der Manipulationsvorrichtung im Körper positioniert wird. Zur Fixierung kann auch ein aufblasbarer Ballon vorgesehen sein.

In Figur 3 (keine Ausführungsform der Erfindung) ist eine endoluminale Ultraschallsonde schematisch abgebildet, bei der die Positioniereinrichtung die Punktionsführung F mit dem Lumen L zur Aufnahme der Manipulationsvorrichtung M und die Ablenkvorrichtung AV als separate Bauteile enthält. Die Ablenkvorrichtung ist am Schaft der Ultraschallsonde bzw. am Schallkopf angebracht. Mit der Steuervorrichtung wird die Ablenkvorrichtung so gesteuert, dass der Austrittswinkel einer in die Punktionsführung vorgeschobenen Diagnose- oder Operationsvorrichtung nach dem Austritt aus der Punktionsführung verändert wird. Die Ablenkvorrichtung kann in der Längsrichtung der Schallsonde oder auch im rechten Winkel zu ihr bewegt werden (siehe Doppelpfeile).

Figur 4 zeigt eine Ultraschallsonde (keine Ausführungsform der Erfindung) bei der die Punktionsführung in den Schaft S der Ultraschallsonde integriert ist. Im Schaft S verläuft neben den Signalleitungen des Ultraschallkopfes K das Lumen L der Punktionsführung.
Die Ablenkvorrichtung AV ist zwischen dem Schaft S und dem Ultraschallkopf K angeordnet und für einen schrägen und/oder axia-len Austritt der Mahipulationsvorrichtung M eingerichtet. Mit der Steuervorrichtung ist die Ablenkvorrichtung AV betätigbar, um den Austrittswinkel oder die axiale Lage der Manipulationsvorrichtung M zu verändern.

Zur Betätigung der Ablenkvorrichtungen AV bei den in den Figuren 1 bis 4 dargestellten Ultraschallsonden kann jeder mechanische, elektromechanische, magnetomechanische oder piezoelektrische Antrieb verwendet werden oder auch eine manuelle Verstellung vorgesehen sein. Derartige Antriebe und Verstellungen sind an sich insbesondere aus der Mikrochirurgie und der Labortechnik allgemein bekannt.

Die Manipulationsvorrichtung ist allgemein eine Untersuchungs- oder Operationsvorrichtung, wie z. B. eine Punktionsnadel, Biopsienadel, Injektionsnadel, ein Katheter oder ein mikrochirurgisches Werkzeug. Gemäß einer besonders vorteilhaften Ausführungsform der Erfindung umfasst die Manipulationsvorrichtung eine Flüssigkeits-Fördereinrichtung, einen Katheter und eine Injektionsnadel. Mit diesem Aufbau ist es möglich, mit der Fördereinrichtung ein Medikament, eine Lösung oder eine Suspension mit biologischen oder biokompatiblen Materialen oder suspendierten Partikeln, wie z. B. lebenden Zellen oder Implantatmaterialien, über den Katheter und die Injektionsnadel ultraschallgezielt in einen bestimmten Gewebebereich oder ein Organ einzuführen. Am Austrittsende des Lumens kann eine integrierte oder als Komponente angesetzte Düse vorgesehen sein. Der Aufbau aus Fördereinrichtung, Katheter und Injektionsnadel kann zur Injektion von Material unter erhöhtem Druck eingerichtet sein (Einpressen von sog. Bulking Agents).

Ein besonderer Vorteil der Erfindung besteht darin, dass mit einer relativ einfach aufgebauten Ablenkvorrichtung, mit der der Winkel des Lumens relativ zum Schaft eingestellt wird, die eingeführte Manipulationsvorrichtung reproduzierbar ausgerichtet werden kann. Besteht die Manipulationsvorrichtung z. B. aus federnd elastischem Edelstahl, so kann dieses in der Ablenkvorrichtung abgewinkelt werde, tritt aber gerade aus dem Lumen aus.

Gemäß abgewandelten, nicht dargestellten Ausführungsformen der Erfindung können an einer Ultraschallsonde mehrere Punktionsführungen vorgesehen sein, die zur gleichzeitigen Führung und Positionierung mehrerer Manipulationsvorrichtungen eingerichtet sind und deren Lage ultraschallgesteuert veränderbar ist. Es können bspw. mehrere Ablenkvorrichtungen auf dem Schaft mehrere röhren- oder schlitzförmige Führungslumen bereitstellen. Alternativ sind diese im Schaft integriert.

Die Manipulationsvorrichtung kann als chirurgisches Arbeitsgerät auch eine Zange, Klemme, Pinzette, Schere und/oder ein Messer aufweisen. Zur Betätigung der Manipulationsvorrichtung ist diese ggf. mit einer externen Antriebsvorrichtung verbunden. Es kann auch eine zusätzliche Antriebsvorrichtung der Punktionsführung vorgesehen sein, die zur Verschwenkung oder Verschiebung der Punktionsführung am Schaft der Ultraschallsonde eingerichtet ist.

Gemäß einer weiteren bevorzugten Ausführungsform der Erfindung ist die Ultraschallsonde mit einem Endoskopiegerät ausgestattet. Das Endoskopiegerät wird als Manipulationsvorrichtung mit der Punktionsführung eingestellt. Alternativ ist das Endoskopiegerät als gesondertes Bauteil an der Ultraschallsonde vorgesehen. Schließlich kann die Manipulationsvorrichtung selbst auch durch eine Sonde mit einem Ultraschallkopf gebildet werden.

Ein besonderer Vorteil der Erfindung besteht darin, dass die Punktionsführung einen relativ einfachen Aufbau besitzt. Dies ermöglicht, dass der Griff, Schaft, Ultraschallkopf und/oder die Punktionsführung sämtlich oder teilweise als Einwegkomponenten hergestellt sind.

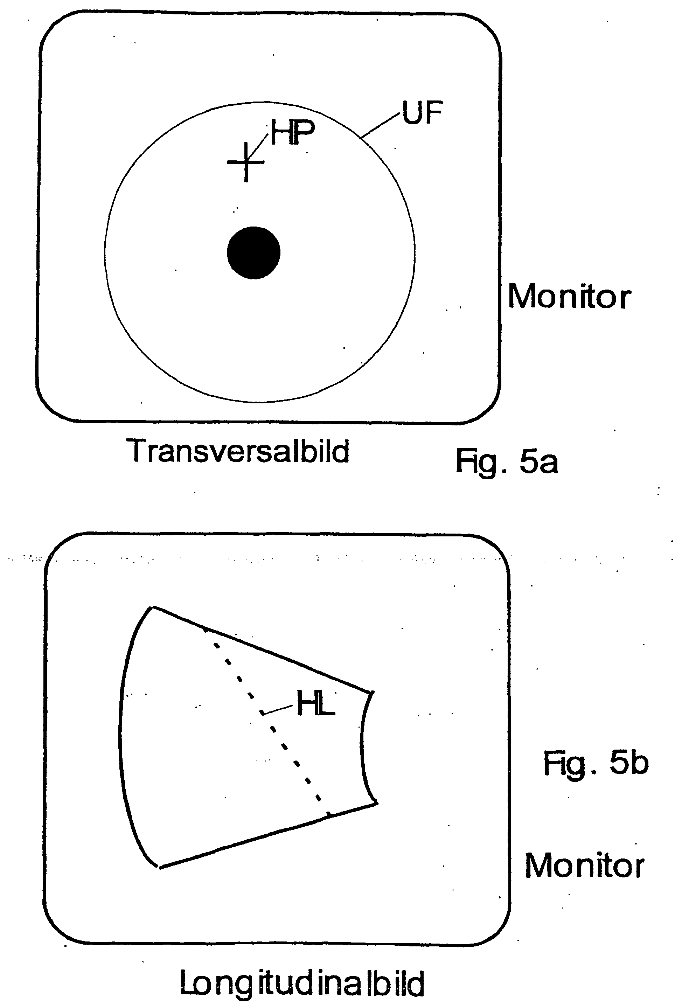

Ein wesentliches Merkmal der Erfindung besteht darin, dass die Manipulationsvorrichtung mit der Punktionsführung in definierter Weise relativ zum Ultraschallkopf positioniert ist oder bewegt wird. Aus den Einstellungen der Punktionsführung und den geometrischen Eigenschaften der Manipulationsvorrichtung können die Relativkoordinaten in Bezug auf den Ultraschallkopf zur Umsetzung der erfindungsgemäßen Anzeige einer Punktionshilfsmarkierung im bildgebenden System der Ultraschallsonde verwendet werden. Dies ist in den Figuren 5a und 5a illustriert.

Figur 5a zeigt in einer schematischen Abbildung eines Monitors M des bildgebenden Systems ein Transversalbild, das von der endoluminalen Ultraschallsonde geliefert wird. In diesem Bild ist als Punktionshilfsmarkierung ein Punktionshilfspunkt HP abgebildet, der vom Koordinatengeber der Steuervorrichtung zur Ansteuerung der Ablenkvorrichtung erzeugt wird. Die Lage des Punktionshilfspunktes HP relativ zum Ultraschallfeld UF wird auf dem Monitor auf der Grundlage von Positionsdaten eingestellt, die die Steuervorrichtung entsprechend der aktuellen Ausrichtung der Positioniereinrichtung liefert. Dadurch kann die Lage einer in die Punktionsführung vorgeschobenen Diagnose- oder Operationsvorrichtung schon vor dem Austritt aus der Punktionsführung im Ultraschallbild erkannt und gezielt verändert werden. In Figur 5b ist als Punktionshilfsmarkierung eine Punktionshilfslinie HL in einem von der Ultraschallsonde gelieferten Longitudinalbild dargestellt. Wiederum kann die Lage einer in die Punktionsführung vorgeschobenen Diagnose- oder Operationsvorrichtung schon vor dem Austritt aus der Punktionsführung im Ultraschallbild erkannt und gezielt verändert werden. Darüber hinaus kann das Vorschieben der Diagnose- oder Operationsvorrichtung im Echtzeit-Ultraschallbild genau verfolgt werden. Die Punktionshilfslinie HL kann auch eine Wegstrecke markieren, die die in der Positioniereinrichtung eingesetzte Manipulationsvorrichtung im vorgeschobenen Zustand bei entsprechender Betätigung der Ablenkvorrichtung zurücklegen würde.

Die Injektion von lebenden Zellen in einem Trägermedium und/oder Biomaterialien oder biokompatiblen Substanzen in einen Organismus umfasst die folgenden Schritte.

Die Ultraschallsonde wird, vorzugsweise in Hohlorgane, wie zum Beispiel den Magen-Darm-Trakt, den Urogenital-Trakt, der Nase und Nasennebenhöhlen, den Atemwege, Gelenke, Körperhöhle, wie z. B. den Bauchraum, den Brustraum, die Schädelhöhle, den Beckenraum, in einen Punktionskanal, Gefäße oder intraoperativ in ein Operationsgebiet eingeführt. Die Einführung erfolgt alternativ mit oder ohne eingesetzter Manipulationsvorrichtung, die hier eine Injektionsvorrichtung ist. Anschließend wird die Positioniereinrichtung, ggf. unter Verwendung der Punktionshilfsmarkierung, eingestellt und ggf. die Injektionsvorrichtung eingesetzt. Die Injektionsvorrichtung ist mit einem Reservoir der zu injizierenden Substanz verbunden. Eine bevorzugte Anwendung ist die Injektion von Medikamenten, Flüssigkeiten, Lösungen, Suspensionen mit biologischen oder biokompatiblem Material und/oder Suspensionen mit lebenden Zellen, insbesondere mit oder ohne Trägermaterialien. Es erfolgt beispielsweise eine transurethale oder transrektale Injektion zur Therapie von Harninkontinenz oder Stuhlinkontinenz. Hierzu werden Myoblasten, Bulking Agents, z. B. Kollagene, pharmakologische Substanzen und/oder Wachstumsfaktoren in den interessierenden Gewebebereich injiziert. Die Myoblasten werden allein oder in Kombination mit biokompatiblen Materialien (Bulking Agents) als Stoffimplantate verwendet. Unter Ultraschallkontrolle wird die Injektion überwacht.

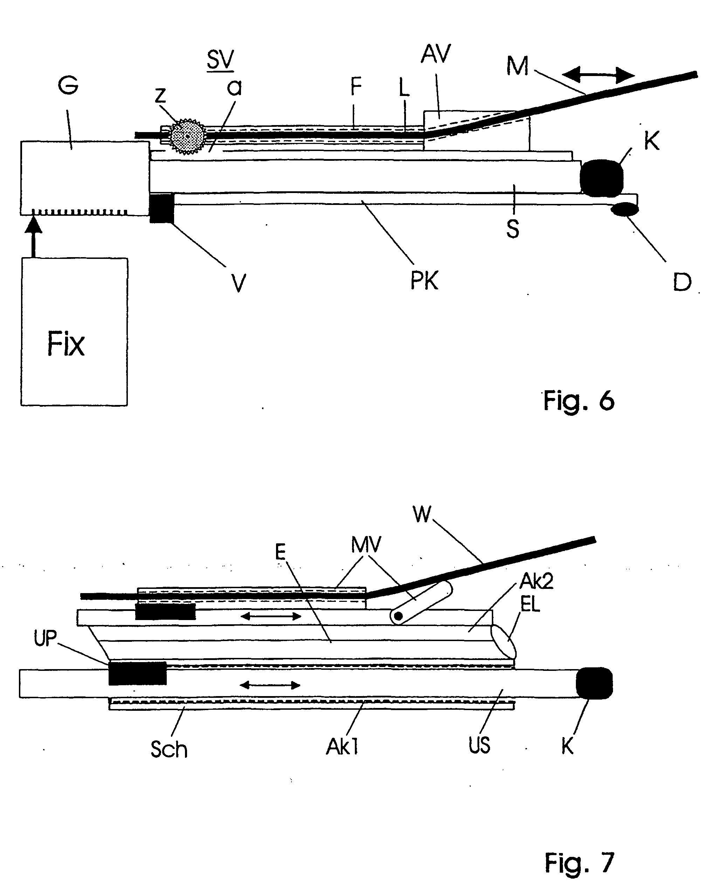

Fig. 6 illustriert eine weitere Ausführungsform der erfindungsgemäßen Ultraschallsonde, die mit einer Ablenkvorrichtung AV für eine Manipulationsvorrichtung M entsprechend einer der oben genannten Gestaltungen ausgestattet ist und die zusätzlich einen Perfusionskanal (oder Perfusionskatheter) PK aufweist. Die Bestandteile der Ultraschallsonde sind, soweit sie analog zu den oben genannten Ausführungsformen offenbart sind, mit den gleichen Bezugszeichen wie in den Fig. 1 bis 4 versehen. Die Abl'enkvorrichtung AV ist dahingehend modifiziert, dass der Vortrieb der Manipulationsvorrichtung M unter Verwendung eines Zahnrads Z erfolgt, das mit einer entsprechenden Zahnstrecke a zusammenwirkt. Der Perfusionskanal PK verläuft parallel zum Schaft S. Das freie Ende des Perfusionskanals PK befindet sich vor oder neben dem Ultraschallkopf K und ist so gestaltet, dass eine von einem externen Reservoir (nicht dargestellt) zugeführte Perfusionsflüssigkeit vor oder neben dem Schallkopf K in das jeweils untersuchte Lumen, z. B. in die Harnröhre in Richtung Blase, austritt, so dass das Lumen mit der Perfusionsflüssigkeit gespült werden kann. Mit der Perfusionsflüssigkeit wird das Lumen vor dem Ultraschallkopf K aufgeweitet. Die Wand des Lumens, z. B. die Wand der Harnröhre, hebt sich vom Ultraschallkopf K ab. Als Ultraschallkopf K wird ein biplanarer Schallkopf verwendet, der zur Aufnahme von Ultraschallbildern in zwei Schallebenen eingerichtet ist. Die erste Schallebene dient der seitlichen, radial gerichteten Betrachtung des Lumens. Die zweite Schallebene dient der axialen Betrachtung des Lumens. Die zweite (oder longitudinale) Schallebene ermöglicht eine Abbildung des von der Perfusionsflüssigkeit geöffneten Raumes vor dem Schaltkopf. Dies stellt einen erheblichen Vorteil gegenüber den herkömmlichen endoskopischen Geräten dar. Die Sonde kann ohne Einsatz eines zusätzlichen Endoskops (wie es z. B. bei der Gestaltung gemäß Fig. 7 vorgesehen ist) gefahrlos in das Lumen, insbe-sondere die Harnröhre, eingeschoben werden. Die Verletzungsgefahr bei transurethraler Sonographie wird deutlich verringert.

Der Perfusionskanal PK ist am entgegengesetzten Ende mit einem Ventil V zum Anschluss einer Flüssigkeitszufuhr ausgestattet.
Das Ventil V wird vorzugsweise so betätigt, dass mit der erfindungsgemäßen endoluminalen Schallsonde das Lumen der Harnröhre mit einem permanenten Perfusionsspülstrom aufgefüllt wird. Bei der Gestaltung der Ultraschallsonde mit dem Perfusionskanal PK stellt die Ultraschallsonde ein Ultraschallzystoskop dar.

Die in Fig. 6 dargestellte Komponente "Fix" stellt eine ortsfeste Fix-Einheit dar, die der Durchführung der an sich bekannten Rückzugs- oder Pull-Back-Technik dient. Mit der erfindungsgemäßen Schallsonde können beim Pull-Back-Verfahren die Querschnittsultraschallbilder beim Zurückziehen (oder analog beim Vorschieben) der Schallsonde mit Hilfe einer planimetrischen Kalkulationsprozedur addiert und somit die gesamte Harnröhrenwand dreidimensional rekonstruiert werden. Dabei ist eine Messeinrichtung mit einem Sensorsystem (siehe Skalierung), das die Relativkoordinaten der Schallsonde in Bezug auf einen Fixpunkt Fix ermittelt, der bei der gesamten Untersuchung in seiner Lage fix am Patienten angebracht ist, vorgesehen. Mit diesem Sensorsystem kann das Verschieben der Schallsonde in der Harnröhre gesteuert und die Relativkoordinaten in die Recheneinheit übertragen werden. Aus den einzelnen Querschnittsbildern wird dann ein dreidimensionales Modell der Harnröhre errechnet.

Am freien Ende des Perfusionskanals PK ist ein Drucksensor und/oder eine Elektrodeneinrichtung D vorgesehen. Es ist an sich bekannt, mit einem Druckmesser das Druckprofil in einem Lumen, z.B. der Harnröhre, zu bestimmen. Erfindungsgemäß wird dies jedoch mit besonderem Vorteil mit der Ultraschallsonde durchgeführt, die mit der Manipulations- und insbesondere Injektionsvorrichtung ausgestattet ist. Eine solche endoluminale Schallsonde gestattet nicht nur die ultraschallgezielte Injektion von Füllstoffen in die Wand der Harnröhre, sondern auch die Kontrolle des Urethradruckprofiles (und damit des Therapieerfolges) mit der selben Vorrichtung während der Therapie.

Die Elektrodeneinrichtung umfasst beispielsweise eine Reizelektrode D, die in unmittelbarer Nähe des Schallkopfs lokalisiert ist und die Feldreizung von Muskelfasern in der Umgebung der Harnröhre und die Erfassung von Kontraktilitätsparametern im Ultraschallbild ermöglicht. Es ist an sich bekannt, dass elektrische Muskelaktivität mit Hilfe von Elektroden, die auf die Oberfläche der Fasern aufgelegt wird, gemessen werden kann (Elektromyogramm). Die erfindungsgemäße transluminale Ultraschallsonde, die mit einer derartigen Elektrode D ausgestattet ist, ermöglicht die Erfassung von elektrischer Muskelaktivität simultan zum Ultraschallbild. Damit können, besonders bei der Diagnostik der Harninkontinenz, wichtige Parameter über die Muskelfunktion in der Harnröhre gewonnen werden.

Mit Hilfe der sogenannten Ultraschallelastographie (siehe Ponnekanti et al. in "Ultrasound in Med. Biol." Bd. 21, 1995, S. 533-543, und Varghese et al. in "Ultrasound in Med. Biol." Bd: 21, 2000, S. 533-543) kann die Elastizität bzw. Rigidität des untersuchten Gewebes bestimmt werden. Bei diesem Verfahren wird mit Hilfe eines Bewegungselementes auf das zu untersuchende Gewebe ein mechanischer Impuls übertragen. Dieses Bewegungselement kann eine Vibrationsvorrichtung in der Sonde sein oder auch eine Verschiebevorrichtung, die mit einem Fixpunkt Fix verbunden ist, der vom zu untersuchenden Objekt (Patienten) unabhängig ist. Vor und nach der definierten mechanischen Kompression des zu untersuchenden Gewebes werden aus den Ultraschallbildern Parameter zur Steifigkeit oder Elastizität des Gewebes ermittelt.

In einer vorteilhaften Ausführungsform der Schallsonde wird die Ultraschallelastographie für die Diagnosestellung bei Harninkontinenz durch Messung der Elastizität der Harnröhre verwendet. Die in Figur 6 dargestellte Schallsonde wird bei der Ultraschall-elastographischen Messung in der Harnröhre definiert verschoben, sodass auf die Harnröhrenwand definierte Dehnungs- und Relaxationskräfte ausgeübt werden. Die Reaktion der Harnröhrenwand auf diese mechanischen Kräfte kann nun von der Schallsonde gemessen und mit Hilfe der Ultraschall-Elastographie quantifiziert werden.

Ein medizinisches Arbeitsgerät ist auszugsweise in Figur 7 illustriert (keine Ausführungsform der Erfindung). Das Arbeitsgerät kann mit besonderem Vorteil zur Injektion einer Suspensionszusammensetzung verwendet werden, die in der unveröffentlichten Patentanmeldung DE 101 19 522.2 beschrieben ist und deren Offenbarungsgehalt in die vorliegende Patentanmeldung einbezogen wird. Das Arbeitsgerät besitzt einen Schaft Sch mit mindestens zwei Arbeitskanälen AK1, AK2. Im Arbeitskanal AK1 ist eine Ultraschallsonde US mit einem Ultraschallkopf K angeordnet. Gemäß einer bevorzugten Ausführungsform der Erfindung ist die Ultraschallsonde US relativ zum Schaft Sch axial beweglich. Eine Messvorrichtung UP ist am Schaft Sch vorgesehen, mit der die Position der Ultraschallsonde US im Arbeitskanal relativ zum Schaft Sch ermittelt werden kann. Die Position der Sonde wird beispielsweise durch Markierungen auf dem Sondenschaft detektiert. Von den Markierungen werden Koordinaten abgeleitet, die dazu benutzt werden können, Längsschnitte der Ultraschallbilder zu räumlichen Bildern zu kombinieren. Im zweiten Arbeitskanal AK2 ist eine Endoskopeinrichtung E angeordnet, an deren körperseitigen Ende beispielsweise eine Endoskoplinse EL zur integrierten Beleuchtung des jeweils untersuchten oder behandelten Gewebes vorgesehen ist. Die Beleuchtung erfolgt vorzugsweise über einen Winkelbereich von 0 bis 90°.

Am zweiten Arbeitskanal AK2 und/oder der Endoskopeinrichtung E ist eine Manipulationsvorrichtung MV zur Betätigung eines Werkzeuges W vorgesehen. Das Werkzeug ist beispielsweise ein Katheter oder eine Spritzennadel. Die Betätigung des Werkzeuges erfolgt in der oben beschriebenen Weise. Das Werkzeug W ist beispielsweise eine Spritzennadel, die Teil der oben beschriebenen Misch- und Applikationsvorrichtungen oder Flüssigkeitsfördervorrichtungen ist.

Die Anwendung des Arbeitsgerätes gemäß Figur 7 erfolgt derart, dass das Arbeitsgerät zunächst im zu behandelnden Körperteil (z. B. Hohlorgan, Gewebe) in Position gebracht wird. Mit der Endoskopeinrichtung E wird das zu behandelnde Gewebe visuell beobachtet. Mit einer Steuervorrichtung oder unter Ultraschallkontrolle wird das Werkzeug W positioniert. Anschließend erfolgt die Applikation der Suspensionszusammensetzung über das Werkzeug W in den interessierenden Gewebebereich, wobei die Koordinaten (insbesondere Endkoordinaten) in der oben beschriebenen Weise bestimmt und der Fortschritt der Applikation mit der Ultraschallsonde US überwacht werden.

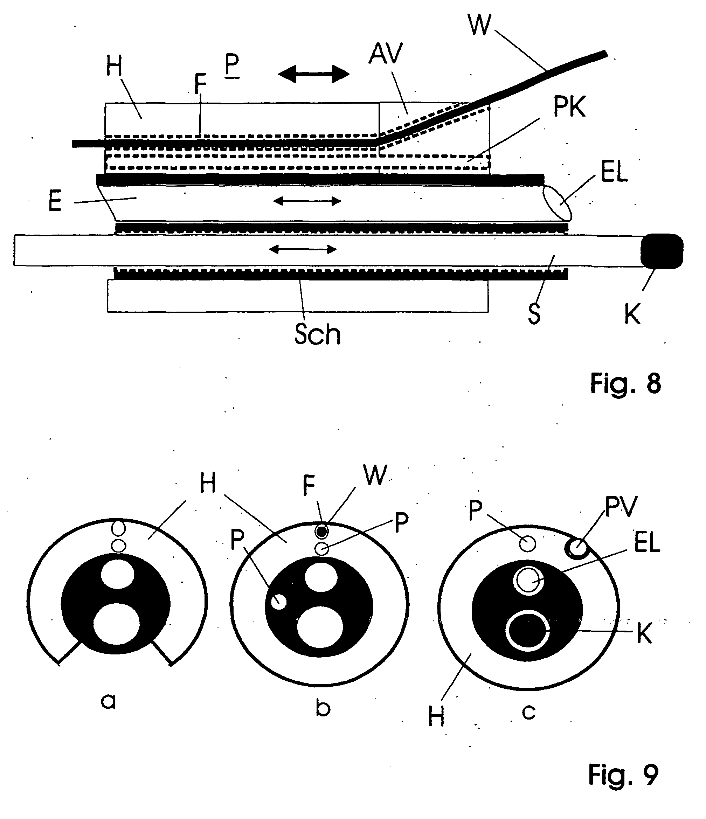

Abwandlungen des in Figur 7 dargestellten Aufbaus sind in der Figur 8 illustriert. Gemäß Figur 8 ist ein Schaft Sch mit zwei Arbeitskanälen vorgesehen, in denen jeweils eine Ultraschallsonde mit einem Sondenkopf K und eine Endoskopeinrichtung E mit einer Endoskoplinse EL axial verschiebbar (Doppelpfeil) angeordnet sind. Auf den Schaft Sch ist außen eine Aufsatzhülse H aufgesteckt, die ein separates und vom Schaft Sch trennbares Bauteil bildet. Die Aufsatzhülse H bildet eine Positioniereinrichtung P mit einer Ablenkvorrichtung AV für das Werkzeug W, das in einer Funktionsführung F angeordnet ist. In der Aufsatzhülse ist des Weiteren der Perfusionskanal PK angeordnet. Die Aufsatzhülse erstreckt sich vorzugsweise über die gesamte Länge des Schaftes Sch, so dass das Ende des Werkzeugs W in der Nähe des Beobachtungsfeldes der Untersuchungseinrichtungen in den Arbeitskanälen angeordnet ist. Die Positioniereinrichtung P ist lösbar mit dem Schaft verbunden.

Figur 9 illustriert die Anordnung der Aufsatzhülse H auf dem Schaft Sch. Die Aufsatzhülse H kann den Schaft Sch teilweise (Fig. 9a) oder vollständig (Figuren 9b, c) umschließen. Im ersten Fall ergibt sich die Möglichkeit eines radial seitlichen Aufsteckens der Aufsatzhülse H, während in den anderen Fällen ein axiales Aufschieben erforderlich ist. Im Schaft Sch sind mindestens zwei Arbeitskanäle vorgesehen. Die Aufsatzhülse H enthält den Perfusionskanal P und die Funktionsführung F mit dem Werkzeug W. Ein zusätzlicher Perfusionskanal P kann auch im Schaft Sch vorgesehen sein (Fig. 9c). Die Aufsatzhülse H kann anwendungsabhängig mit zusätzlichen, nach außen offenen Kanälen ausgestattet sein, in die mindestens eine zusätzliche Perfusionsvorrichtung PV einsetzbar ist. Die Perfusionsvorrichtung PV ist insbesondere für eine radiale Spülung des Bereiches im Organismus vorgesehen, in den das Arbeitsgerät eingeführt ist. Die Perfusionsvorrichtung PV bildet z. B. einen abnehmbaren Katheder, der in einer U-förmigen Vertiefung in der Aufsatzhülse H oder auch im Schaft des Arbeitsgerätes verläuft.

Die Aufsatzhülse H kann auf dem Schaft Sch verschoben und/oder gedreht werden, insbesondere um das Werkzeug W exakt auszurichten.

## Patentansprüche

1. Ultraschallsonde mit:
- einem Ultraschallkopf (K), der an einem starren Schaft (S) befestigt und mit diesem beweglich ist, und
- einer Positioniereinrichtung (P) für eine Untersuchungs- oder Operationsvorrichtung (M), wobei
- die Positioniereinrichtung (P) auf der Außenseite des Schaftes (S) angeordnet ist und eine lösbar mit dem Schaft (S) verbundene Punktionsführung (F) mit einem rohr- oder schlitzförmigen Lumen (L) zur Aufnahme der Untersuchungs- oder Operationsvorrichtung (M) aufweist, wobei
- die Punktionsführung (F) um eine vorbestimmte Strecke parallel zur Längsachse des Schaftes (S) verschiebbar ist,
- das Lumen (L) einen vorbestimmten Austrittswinkel relativ zur Längsachse des Schaftes (S) aufweist, und
- die Punktionsführung (F) ein langgestrecktes Bauteil bildet, das parallel zur Längsachse des Schaftes (S) verläuft und für die Untersuchungs- oder Operationsvorrichtung (M) eine Ablenkvorrichtung (AV) bildet,
**dadurch gekennzeichnet, dass**
in der Ablenkvorrichtung (AV) das Lumen (L) einen relativ zur Längsachse des Schaftes (S) gekrümmten Verlauf besitzt.

2. Ultraschallsonde gemäß einem der vorhergehenden Ansprüche, bei der die Ablenkvorrichtung (AV) mit einer Steuervorrichtung (SV) betätigbar ist, die mit einer Recheneinheit zur Ermittlung von Relativkoordinaten des Lumens, der Austrittsöffnung des Lumens oder der im Betriebszustand in das Lumen eingeführten Untersuchungs- oder Operationsvorrichtung (M) in Bezug auf den Ultraschallkopf (K) und einem Koordinatengeber verbunden ist.

3. Ultraschallsonde gemäß Anspruch 2, die mit einem bildgebenden System und einer Anzeigeinrichtung zur Darstellung eines Ultraschallbildes ausgestattet ist, wobei der Koordinatengeber mit dem bildgebenden System und der Anzeigeeinrichtung verbunden und dazu eingerichtet ist, Bilddaten an das bildgebende System zu übergeben, die charakteristisch für die von der Recheneinheit ermittelten Relativkoordinaten sind.

4. Ultraschallsonde gemäß einem der vorhergehenden Ansprüche, bei der die Punktionsführung (F) aus sterilisierbarem und wiederverwertbarem Material oder aus Einwegmaterial besteht.

5. Ultraschallsonde gemäß Anspruch 4, bei der die Punktionsführung (F) aus Kunststoff besteht.

6. Ultraschallsonde gemäß einem der vorhergehenden Ansprüche, die mit einem Endoskop ausgestattet ist.

7. Ultraschallsonde gemäß einem der vorhergehenden Ansprüche, bei der am Ende des Lumens (L) eine Austrittsdüse vorgesehen ist.

8. Ultraschallsonde gemäß einem der vorhergehenden Ansprüche, die mit einem Perfusionskanal oder Perfusionskatheter mit einem freien Ende neben oder vor dem Ultraschallkopf (K) ausgestattet ist.

9. Ultraschallsonde gemäß einem der vorhergehenden Ansprüche, die am freien Ende des Schaftes (S) mit einem Drucksensor (D) und/oder einer Vibrations- oder Verschiebevorrichtung ausgestattet ist.

10. Ultraschallsonde gemäß einem der vorhergehenden Ansprüche, die mit einer Elektrodeneinrichtung mit mindestens einer Reizelektrode und/ oder mindestens einer Elektromyogramm-Elektrode (D) ausgestattet ist.

11. Ultraschallsonde gemäß einem der vorhergehenden Ansprüche, die mindestens einen lösbaren Haken und/oder mindestens einen aufblasbaren Ballon aufweist, mit dem der Ultraschallkopf (K), die Positioniereinrichtung und/oder der Schaft (S) an einem zu untersuchenden oder zu behandelnden Gewebe oder Organ lösbar fixiert werden kann.

12. Medizinisches Gerät, das eine Ultraschallsonde gemäß einem der vorhergehenden Ansprüche und mindestens eine in der Positioniereinrichtung angeordnete Untersuchungs- oder Operationsvorrichtung (M) aufweist.

13. Medizinisches Gerät gemäß Anspruch 12, bei dem die Untersuchungs- oder Operationsvorrichtung eine Biopsienadel, eine Pipette, einen Katheter, eine Injektionsnadel, ein chirurgisches Arbeitsgerät, eine Arbeitssonde, vorzugsweise eine Laser-, Koagulations-, Hochfrequenz-, Radiofrequenz- oder Kryosonde, eine Ultraschallsonde und/oder ein Endoskop umfasst.

14. Medizinisches Gerät gemäß Anspruch 13, das mit einem hydropneumatischen System ausgestattet ist, mit dem durch das Lumen (L) oder durch eine in das Lumen (L) eingeführte Düse Medikamente, Lösungen, Suspensionen und/oder lebende Zellen spritzbar sind.

## Claims

1. Ultrasonic probe comprising:
- an ultrasonic head (K) which is secured to a rigid shaft (S) and moveable with this shaft, and
- a positioning apparatus (P) for an examination or operation device (M),
- the positioning apparatus (P) being arranged on the outside of the shaft (S) and having a puncture guide (F) which is detachably connected to the shaft (S) and has a tubular or slot-shaped lumen (L) for receiving the examination or operation device (M),
- the puncture guide (F) being displaceable at a predetermined distance parallel to the longitudinal axis of the shaft (S),
- the lumen (L) having a predetermined exit angle relative to the longitudinal axis of the shaft (S), and
- the puncture guide (F) forming an elongated component which extends parallel to the longitudinal axis of the shaft (S) and forms a deflection device (AV) for the examination or operation device (M),
**characterised in that**
in the deflection device (AV), the lumen (L) has a curved course relative to the longitudinal axis of the shaft (S).

2. Ultrasonic probe according to one of the preceding claims, in which the deflection device (AV) may be actuated by means of a control device (SV) which is connected to a calculating unit for determining relative coordinates of the lumen, of the exit aperture of the lumen or of the examination or operation device (M) inserted into the lumen in an operating state, with reference to the ultrasonic head (K) and a coordinate generator.

3. Ultrasonic probe according to claim 2, which is equipped with an image-generating system and a display device for showing an ultrasonic image, the coordinate generator being connected to the image-generating system and the display device and being arranged for transferring image data to the image-generating system which are characteristic for the relative coordinates determined by the calculating unit.

4. Ultrasonic probe according to one of the preceding claims, in which the puncture guide (F) consists of sterilisable and reusable material or of disposable material.

5. Ultrasonic probe according to claim 4, in which the puncture guide (F) consists of plastics material.

6. Ultrasonic probe according to one of the preceding claims, which is equipped with an endoscope.

7. Ultrasonic probe according to one of the preceding claims, in which an exit nozzle is provided at the end of the lumen (L).

8. Ultrasonic probe according to one of the preceding claims, which is equipped with a perfusion channel or perfusion catheter with a free end near or in front of the ultrasonic head (K).

9. Ultrasonic probe according to one of the preceding claims, which is equipped at the free end of the shaft (S) with a pressure sensor (D) and/or a vibration or displacement device.

10. Ultrasonic probe according to one of the preceding claims, which is equipped with an electrode device having at least one stimulus electrode and/or at least one electromyogram electrode (D).

11. Ultrasonic probe according to one of the preceding claims, which has at least one detachable hook and/or at least one inflatable balloon with which the ultrasonic head (K), the positioning apparatus and/or the shaft (S) can be detachably fixed to a tissue or organ to be examined or treated.

12. Medical apparatus which has an ultrasonic probe according to one of the preceding claims and at least one examination or operation device (M) arranged in the positioning apparatus.

13. Medical apparatus according to claim 12, in which the examination or operation device includes a biopsy needle, a pipette, a catheter, an injection needle, a surgical implement, a working probe, preferably a laser, coagulation, high-frequency, radio-frequency probe or a cryoprobe, an ultrasonic probe and/or an endoscope.

14. Medical apparatus according to claim 13, which is equipped with a hydro-pneumatic system with which pharmaceutical products, solutions, suspensions and/or living cells may be sprayed through the lumen (L) or through a nozzle inserted into the lumen (L).

## Revendications

1. Sonde à ultrasons avec :
- une tête à ultrasons (K) qui est fixée à une tige rigide (S) et qui est mobile avec cette dernière, et
- un système de positionnement (P) destiné à un dispositif d'exploration ou d'intervention (M), moyennant quoi
- le système de positionnement (P) est disposé sur la face extérieure de la tige (S) et présente un guidage de ponction (F) amovible relié à la tige (S) avec un lumen (L) en forme de tube ou de fente pour loger le dispositif d'exploration ou d'intervention (M), moyennant quoi
- le guidage de ponction (F) peut être déplacé sur un trajet prédéfini parallèlement à l'axe longitudinal de la tige (S),
- le lumen (L) présente un angle de sortie prédéfini par rapport à l'axe longitudinal de la tige (S), et
- le guidage de ponction (F) constitue un élément de structure étiré en longueur, qui s'étend parallèlement à l'axe longitudinal de la tige (S) et constitue un dispositif de déviation (AV) pour le dispositif d'exploration ou d'intervention (M),
**caractérisé en ce que**
- dans le dispositif de déviation (AV) le lumen (L) présente un parcours courbe par rapport à l'axe longitudinal de la tige (S).

2. Sonde à ultrasons selon l'une quelconque des revendications précédentes, dans laquelle le dispositif de déviation (AV) peut être actionné avec un dispositif de commande (SV), lequel est relié à une unité de calcul pour calculer des coordonnées relatives du lumen, l'ouverture de sortie du lumen ou du dispositif d'exploration ou d'intervention (M) introduit en fonctionnement dans le lumen par rapport à la tête à ultrasons (K) et relié également à un donneur de coordonnées.

3. Sonde à ultrasons selon la revendication 2, dotée d'un système fournissant une image et un système d'affichage pour représenter une image à ultrasons, moyennant quoi le donneur de coordonnées est relié au système fournissant une image et au système d'affichage et est réglé de façon à transmettre au système fournissant une image des données d'image, qui sont caractéristiques pour les coordonnées relatives calculées par l'unité de calcul.

4. Sonde à ultrasons selon l'une quelconque des revendications précédentes dans laquelle le guidage de ponction (F) est composé de matériau stérilisable et recyclable ou de matériau jetable.

5. Sonde à ultrasons selon la revendication 4, dans laquelle le guidage de ponction (F) est en matière plastique.

6. Sonde à ultrasons selon l'une quelconque des revendications précédentes dotée d'un endoscope.

7. Sonde à ultrasons selon l'une quelconque des revendications précédentes dans laquelle est prévu à l'extrémité du lumen (L) une buse de sortie.

8. Sonde à ultrasons selon l'une quelconque des revendications précédentes dotée d'un canal de perfusion ou d'un cathéter de perfusion avec une extrémité libre près de ou avant la tête à ultrasons (K).

9. Sonde à ultrasons selon l'une quelconque des revendications précédentes dotée à l'extrémité libre de la tige (S) d'un capteur de pression (D) et/ou d'un dispositif de vibration ou de déplacement.

10. Sonde à ultrasons selon l'une quelconque des revendications précédentes dotée d'un dispositif à électrodes avec au moins une électrode d'excitation et/ou au moins une électrode d'électromyogramme (D).

11. Sonde à ultrasons selon l'une quelconque des revendications précédentes qui présente au moins un crochet amovible et/ou au moins un ballon gonflable avec lequel la tête à ultrasons (K), le système de positionnement et/ou la tige (S) peuvent être fixés de manière amovible à un tissu ou à un organe à explorer ou à traiter.

12. Appareil médical qui présente une sonde à ultrasons selon l'une quelconque des revendications précédentes et au moins un dispositif d'exploration ou d'intervention (M) disposé dans le système de positionnement.

13. Appareil médical selon la revendication 12, dans lequel le dispositif d'exploration ou d'intervention comprend une aiguille pour biopsie, une pipette, un cathéter, une aiguille pour injection, un appareil de travail chirurgical, une sonde de travail, de préférence une sonde laser, une sonde à coagulation, une sonde à haute fréquence, une sonde à fréquence radio ou une cryosonde, une sonde à ultrasons et/ou un endoscope.

14. Appareil médical selon la revendication 13, doté d'un système hydropneumatique, avec lequel des médicaments, des solutions, des suspensions et/ou des cellules vivantes peuvent être injectées grâce au lumen (L) ou grâce à une buse introduite dans le lumen (L).
